# EUROPÄISCHE PATENTSCHRIFT

(11) **EP 1 002 546 B1**
(45) Veröffentlichungstag und Bekanntmachung des Hinweises auf die Patenterteilung: **24.08.2005**
(21) Anmeldenummer: 99121809.0
(22) Anmeldetag: 04.11.1999
(51) Int. Cl.: A61K 47/14, A61K 47/18, C08G 65/332, C07C 69/675, C07C 69/732, A23L 1/035, A61K 7/48

(54) **Verwendung von Estern der 12-Hydroxystearinsäure als Solubilisatoren**
Use of esters of 12-hydroxystearic acid as solubilizers
Utilisation d'esters d'acide 12-hydroxystéarique comme solubilisateurs

(30) Priorität: 10.11.1998 DE 19851777
(43) Veröffentlichungstag der Anmeldung: 24.05.2000
(73) Patentinhaber: BASF Aktiengesellschaft, 67056 Ludwigshafen (DE)
(72) Erfinder: Zirnstein, Michael, Dr., 69198 Schriesheim (DE); Ruchatz, Folker, Dr., 67433 Neustadt (DE); Kolter, Karl, Dr., 67117 Limburgerhof (DE); Oppenländer, Knut, Dr., 67061 Ludwigshafen (DE)

(56) Entgegenhaltungen:
- EP-A- 0 031 310
- WO-A-97/10849
- WO-A-99/02168
- DE-A- 4 331 228
- US-A- 4 745 160
- DATABASE WPI Week 198422 Derwent Publications Ltd., London, GB; AN 1984-136506 XP002239885 & JP 59 069135 A (NIPPON OILS & FATS CO. LTD.), 19. April 1984 (1984-04-19)
- LORENZ W ET AL: "Histamine release and hypotensive reactions in dogs by solubilizing agents and fatty acids: Analysis of various components in cremophor E1 and development of a compound with reduced toxicity" AGENTS AND ACTIONS 1982 SWITZERLAND, Bd. 12, Nr. 1-2, 1982, Seiten 64-80, XP009009713

## Beschreibung

Die Erfindung betrifft die Verwendung von Estern der 12-Hydroxystearinsäure als Solubilisator.

Bei der Herstellung homogener pharmazeutischer oder kosmetischer Zubereitungen hat die Solubilisierung von hydrophoben Stoffen eine sehr große praktische Bedeutung erlangt.

Unter Solubilisierung ist eine Löslichkeitsverbesserung durch oberflächenaktive Verbindungen zu verstehen, die in der Lage sind, schlecht wasserlösliche oder wasserunlösliche Stoffe in klare, höchstens opaleszierende wäßrige Lösungen zu überführen, ohne daß hierbei die chemische Struktur dieser Stoffe eine Veränderung erfährt.

Die hergestellten Solubilisate sind dadurch gekennzeichnet, daß der schlecht wasserlösliche oder wasserunlösliche Stoff in den Molekülassoziaten der oberflächenaktiven Verbindungen, die sich in wäßriger Lösung bilden - den sogenannten Mizellen - gelöst vorliegt. Die resultierenden Lösungen sind stabile einphasige Systeme, die optisch klar bis opaleszent erscheinen und ohne größeren Energieeintrag hergestellt werden können.

Solubilisatoren können das Aussehen beispielsweise von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich folgende Produkte eingesetzt:
- ethoxiliertes (hydriertes) Ricinusöl, (z.B. Cremophor® Marken, Fa. BASF);
- ethoxilierte Sorbitanfettsäureester, (z.B. Tween® Marken, Fa. ICI);
- ethoxilierte Hydroxystearinsäure, (z.B. Solutol® Marken, Fa. BASF).

Die oben beschriebenen, bisher eingesetzten Solubilisatoren zeigen jedoch eine Reihe anwendungstechnischer Nachteile.

So ist z. B. deren parenterale Applikation mit einer Freisetzung von Histamin und einem daraus resultierenden Blutdruckabfall verbunden (Lorenz et al., Agents and Actions, Vol. 12, 1/2, 1982).

Die bekannten Solubilisatoren besitzen für einige schwerlösliche Arzneistoffe z. B. Clotrimazol nur eine geringe lösungsvermittelnde Wirkung.

Oberflächenaktive Verbindungen besitzen häufig eine hohe hämolytische Aktivität, die einer Anwendung auf dem Gebiet der Pharmazie, insbesondere in Parenteralia entgegensteht.

EP-A-0 017 059 beschreibt die Herstellung von alkoxylierten Fettsäuren vom Typ Solutol® und deren Verwendung als Lösungsvermittler. Solubilisatoren dieses Strukturtyps zeigen jedoch die oben genannten Nachteile.

DE-A-4 331 228 beschreibt die Umsetzung von Polyethylenglykol mit Hydroxycarbonsäuren und die Verwendung dieser Reaktionsprodukte als Entschäumer für wäßrige Systeme.

JP 59069135 beschreibt die Verwendung von Polyoxyalkylenester von Ricinolsäure als Emulgator. Die hier offenbarten Verbindungen haben den Nachteil, daß sie entweder nicht genügend wasserlöslich sind oder bei parenteraler Applikation eine unerwünscht hohe Freisetzung von Histamin hervorrufen.

FR 2056177 beschreibt Wasser-in-Öl Emulsionen mit alkoxylierten Fettsäuren als Emulgatoren.

Es bestand nun die Aufgabe, neue Solubilisatoren für pharmazeutische, kosmetische sowie Lebenmittel-Zubereitungen bereitzustellen, die die oben genannten Nachteile nicht aufweisen.

Diese Aufgabe wurde erfindungsgemäß gelöst durch die Verwendungvon Estern der 12-Hydroxystearinsäure der allgemeinen Formel I, 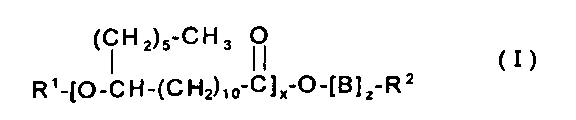 in der die Substituenten und Variablen unabhängig voneinander folgende Bedeutung haben:
R¹ Wasserstoff, C₁₂-C₂₂-Acyl, 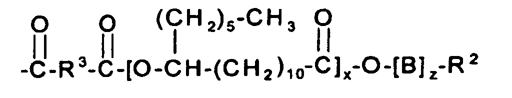
   - R²: Methyl;
   - R³: Rest einer dimerisierten Fettsäure;
   - B: -CH₂-CH₂-O-;
   - x: 1 bis 2;
   - z: 10 bis 18.
   als Solubilisatoren.

Die OH-Gruppen der erfindungsgemäß verwendeten 12-Hydroxystearinsäure können ungeschützt oder acyliert vorliegen.

Von den Acylresten der C₁₂-C₂₂-Carbonsäuren seien vorzugsweise genannt: Laurinsäure, Stearinsäure, Ölsäure, Behensäure; besonders bevorzugt Stearinsäure.

Im Falle hydroxylierter Acylreste seien insbesondere die Reste von 12-Hydroxystearinsäure, Ricinolsäure, besonders bevorzugt 12-Hydroxystearinsäure genannt.

Als geeignete Dihydroxyacylreste seien 9,10-Dihydroxystearinsäurereste genannt.

Zu den geeigneten Trihydroxyacylresten zählen 9,10,12-Trihydroxystearinsäurereste.

Die OH-Gruppen der erfindungsgemäß verwendeten 12-Hydroxystearinsäure können auch an Dicarbonsäuren gebunden sein. Zu den geeigneten Dicarbonsäuren zählen aliphatische Dicarbonsäuren, aromatische Dicarbonsäuren sowie Dimerfettsäuren (polymerisierte Fettsäuren).

Unter dimerisierten Fettsäuren sind gesättigte oder ungesättigte Fettsäuren mit 12 bis 44 C-Atomen, bevorzugt 32 bis 40 C-Atomen zu verstehen, die durch Dimerisierung einer oder verschiedener ungesättigter Fettsäuren hergestellt werden.

Die dimerisierbaren Fettsäuren sind einfach oder mehrfach ungesättigte Verbindungen mit einer Kohlenstoffkette von 6 bis 22 C-Atomen, bevorzugt 12 bis 22 C-Atomen, besonders bevorzugt 16 bis 20 C-Atomen sowie Gemische dieser Fettsäuren bzw. Fettalkohole, beispielsweise Ölsäure - Linolsäure Gemische.

Die dimerisierten Fettsäure enthalten im wesentlichen lineare und cyclische Verbindungen, die ungesättigt oder hydriert sein können, bevorzugt aber hydriert sind.

### Beispiele für ungesättigte Dimerfettsäurestrukturen:

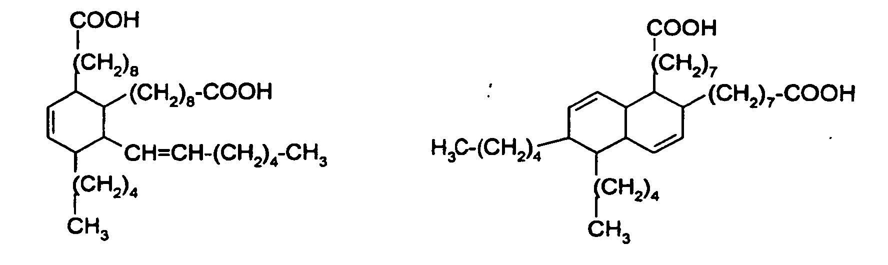

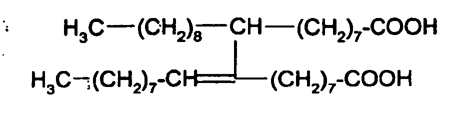

Als dimerisierte Fettsäuren kommen vorzugsweise die Produkte in Frage, die unter der Bezeichnung Pripol® (Fa. Unichema) oder Empol® (Fa. Henkel) im Handel erhältlich sind. Diese dimerisierten Öl-/Linolsäure Gemische enthalten vorwiegend lineare und cyclische Verbindungen. Daneben können diese Produkte auch noch Anteile von monomeren sowie von trimeren und höher kondensierten Fettsäuren enthalten.

Typische im Handel erhältliche dimere Fettsäuren haben etwa folgende Zusammensetzung:

| | |
|---|---|
| Monomere Säuren | 0-15 Gew.-%, |
| dimere Säuren | 50-99 Gew.-%, |
| tri- und höherpolymerisierte Säuren | 1-35 Gew.-%, |

wobei der Gehalt je nach Herkunft der Monomeren, des Polymerisationsverfahrens sowie des Aufarbeitungsprozesses innerhalb dieser Grenzen schwanken kann.

Für die Kondensation mit 12-Hydroxystearinsäure sind verschlossene Methylpolyethylenglykole geeignet, besonders bevorzugt sind Methylpolyethylenglykole mit einem (mittleren) Molekulargewicht von 450 bis 800 g/mol.

Die Verknüpfung der hydrophilen Verbindungen mit 12-Hydroxystearinsäure erfolgt über Esterbindungen

Die Herstellung der bevorzugt verwendeten 12-HydroxystearinsäureEster kann nach an sich bekannten Verfahren durch Kondensation von 12-Hydroxystearinsäure mit den oben genannten hydrophilen Verbindungen erfolgen.

Es können auch Hydroxystearinsäureester wie z.B. Methylester, Ethylester oder entsprechenden Lactone zur Umesterung eingesetzt werden.

Ebenso können entsprechende Säurechloride oder Anhydride (auch gemischte Anhydride) eingesetzt werden.

So kann 12-Hydroxystearinsäure oder gegebenenfalls ein Carbonsäuregemisch bestehend aus 12-Hydroxystearinsäure und zusätzlich einer oder mehrerer der oben genannten Carbonsäuren in einem "Eintopfverfahren" mit der hydrophilen Verbindung zu den erfindungsgemäßen 12-Hydroxystearinsäure-Derivaten kondensiert werden.

Wird neben 12-Hydroxystearinsäure zusätzlich eine oder mehrere der oben beschriebenen Carbonsäuren eingesetzt, so liegt das molare Verhältnis von 12-Hydroxystearinsäure zu den weiteren Carbonsäuren im Bereich von 0,5 zu 1 bis 10 zu 1, bevorzugt im Bereich von 1 zu 1 bis 4 zu 1.

Im Unterschied zu dem "Eintopfverfahren" besteht auch die Möglichkeit zunächst ein Vorkondensat aus Hydroxystearinsäure bzw. aus Hydroxystearinsäure und den weiteren Carbonsäuren herzustellen, und erst dann die Kondensation mit der hydrophilen Polyalkylenverbindung durchzuführen.

Es ist auch möglich zunächst ein Vorkondensat aus Hydroxystearinsäure und der hydrophilen Verbindung herzustellen, das dann mit der weiteren Carbonsäure umgesetzt werden kann.

Die Kondensation kann unter Verwendung eines sauren oder eines basischen Katalysators oder ohne zusätzlichen Katalysator durchgeführt werden.

Als saure Katalysatoren eigenen sich sowohl Brönstedtsäuren als auch Lewissäuren, beispielsweise Schwefelsäure, p-Toluolsulfonsäure, Salzsäure, phosphorige Säure, hypophosphorige Säure, Phosphorsäure, Methansulfonsäure, Borsäure, Aluminiumchlorid, Bortrifluorid, Tetraethylorthotitanat, Zinndioxid, Zinnbutyldilaurat oder deren Gemische. Bevorzugt sind hypophosphorige Säure, phosphorige Säure, Phosphorsäure, p-Toluolsulfonsäure und deren binäre Gemische.

Als basische Katalysatoren eigenen sich Natriummethylat, Natriumethylat, Kaliumcarbonat, Natriumcarbonat, Calciumcarbonat, Magnesiumcarbonat, Kaliumtertiärbutylat, Kaliumhydroxid, Natriumhydroxid, Lithiumhydroxid, Magesiumoxid, Kaliumphosphat, Natriumborhydrid.

Der Katalysator wird dabei in Mengen von 0,05 bis 10 Gew.%, bevorzugt 0,1 bis 5 Gew.%, bezogen auf die Ausgangsstoffe, eingesetzt.

Die Reaktion kann in Lösungsmitteln oder lösungsmittelfrei durchgeführt werden. Bevorzugt ist die lösungsmittelfreie Fahrweise.
Als Lösungsmittel eigenen sich z. B. Toluol, Xylol, Acetonitril, N,N-Dimethylformamid, N,N-Dimethylacetamid, N-Methylpyrrolidon, Diglyme, Dimethylethylenglykol, Tetrahydrofuran, Dioxan, Ethylencarbonat, Propylencarbonat.

Nach Beendigung der Umsetzung oder während der Umsetzung kann das Lösungsmittel abdestilliert werden.

Die Kondensation wird in der Regel bei Drücken von 5 mbar bis Normaldruck und bei Temperaturen von 60 bis 220°C, bevorzugt 120 bis 180°C, besonders bevorzugt bei 130 bis 170°C; im Falle eines 12-Hydroxystearinsäureesters als Edukt bei 30 bis 220°C, bevorzugt bei 60 bis 170°C durchgeführt. Die Reaktionszeiten liegen bei 2 bis 25 Stunden. Die Reaktionskontrolle erfolgt über die Menge an entferntem Reaktionswasser oder über die Bestimmung der Säurezahl bzw. - im Falle eines 12-Hydroxystearinsäureesters als Edukt - über die Menge an betreffendem Alkohol.

Verbindungen der Struktur Ib, 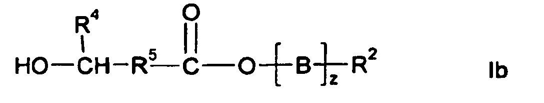 bei der der Substituent R⁴ die Bedeutung -(CH₂)₅-CH₃ hat und der Substituent R⁵ die Bedeutung -(CH₂)₁₀ und die Substituenten R² sowie die Gruppe [B]_{z} die bereits eingangs genannte Bedeutung haben, lassen sich durch gezielte Schutzgruppensynthese herstellen, bei welcher durch gezielte Blockierung der OH-Gruppe der hydroxylierten Carbonsäuren eine Selbstkondensation zu Dimeren, Trimeren sowie weiteren Oligomeren im nachfolgenden Veresterungsschritt mit den einseitig verschlossenen Polyalkylenglykolderivaten verhindert wird.

In einer speziellen Ausführung läßt sich beispielsweise die 12-Hydroxygruppe der 12-Hydroxystearinsäure als THP-Ether gegenüber Veresterung schützen. Dies kann durch Umsetzung von reinem 12-Hydroxystearinsäuremethylester unter p-Toluolsulfonsäure-Katalyse mit Dihydropyran erfolgen. Der gebildete 12-O-Tetrahydropyranyl-12-hydroxystearinsäuremethylester kann anschließend zur entsprechenden Carbonsäure hydrolysiert werden. Die anschließende Veresterung der 12-O-Tetrahydropyranyl-12-hydroxystearinsäure beispielsweise mit Methylpolyglykolen kann in an sich bekannter Weise u.a. unter Katalyse von Dimethylaminopyridin (DMAP) mittels Dicyclohexylcarbodiimid (DCC) erfolgen. Nach Abspaltung der THP-Schutzgruppe lassen sich 12-Hydroxystearinsäuremethylpolyethylenglykol-monoester frei von oligomeren Stearinsäureanteilen erhalten.

Geeignet sind alle Schutzgruppen für Hydroxyfunktionen, die sich möglichst ohne Nebenreaktionen, insbesondere weitgehend ohne Selbstveresterung der 12-Hydroxystearinsäure oder ihrer Derivate einführen lassen, unter den gewählten Veresterungsbedingungen hinreichend stabil sind und sich nach erfolgter Veresterung unter Vermeidung einer Esterspaltung wieder abspalten lassen.

Geeignete Schutzgruppen für Hydroxy-Funktionen sind dem Fachmann bekannt und werden beispielsweise beschrieben von T. W. Greene, P. G. M. Wuts in "Protective Groups in Organic Synthesis", Second Edition, S.14-118, John Wiley Sons, Inc. (1991). Zu diesen Schutzgruppen zählen solche die unter Etherbildung mit der Hydroxygruppe reagieren. Dies sind beispielsweise die Methyl-, Methoxymethyl-, Tetrahydropyranyl-, 2,2,2-Trichloroethyl-, t-Butyl-, Allyl-, Benzyl- und p-Methoxybenzyl-Schutzgruppe; Silyl-Schutzgruppen wie die Trimethylsilyl-, Triisopropylsilyl- und t-Butyldimethylsilyl-Schutzgruppe.

Als Schutzgruppen kommen außerdem solche in Betracht, die unter Esterbildung mit der Hydroxygruppe reagieren. Hierzu zählen Acetat, Chloroacetat, Trichloroacetat, Methylcarbonat, Benzylcarbonat.

Bevorzugte Schutzgruppen sind die Tetrahydropyranyl- und die Benzyl-Schutzgruppe.

### Anwendungen:

Durch die vorliegende Erfindung werden wasserlösliche, amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe in wäßrigen Systemen zu solubilisieren.

Überraschend wurde bei den beanspruchten Verbindungen ein gutes Solubilisationsvermögen für pharmazeutische und kosmetische Wirkstoffe gefunden. Ferner werden mit den beanspruchten Verbindungen Anwendungen erhalten, die sich durch eine sehr geringe Hämolyserate, einer nebenwirkungsfreien Verträglichkeit nach parenteraler, oraler und topischer Applikation auf Haut- und Schleimhaut auszeichnen. Die Verbindungen besitzen insbesondere keine Nebenwirkungen durch Wechselwirkungen mit Blutkörperchenmembranen. Nach parenteraler Applikation findet keine bzw. nur eine geringe Histaminfreisetzung statt. Die Solubilisatoren sind aufgrund ihres geringen Molekulargewichts nierengängig.

Ferner ist zu erwarten, daß mit den erfindungsgemäß verwendeten Carbonsäurederivaten in der pharmazeutischen Anwendung ein "Multi-drug-resistance-reversal" zu erreichen ist.

### Solubilisatoren für Kosmetik

Die Verbindungen der Formel I können als Solubilisatoren in kosmetischen Formulierungen eingesetzt werden. Besonders eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Weiterhin können die erfindungsgemäßen Verbindungen der Formel I als Solubilisatoren für in Wasser schwerlösliche oder unlösliche UV-Absorber wie beispielsweise 2-Hydroxy-4-methoxybenzophenon (Uvinul® M 40, Fa. BASF), 2,2',4,4'-Tetrahydroxybenzophenon (Uvinul® D 50), 2,2'-Dihydroxy-4,4'-dimethoxybenzophenon (Uvinul® D49), 2, 4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul® T 150), 3-(4-Methoxybenzyliden)-campher (Eusolex® 6300, Fa. Merck), N,N-Dimethyl-4-aminobenzoesäure-2-ethylhexylester (Eusolex® 6007), Salicylsäure-3,3,5-trimethylcyclohexylester, 4-Isopropyl-dibenzoylmethan (Eusolex® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens eine der Verbindungen der Formel I als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol-Basis. Die Verbindung I wird als Solubilisator im Verhältnis von 0,2:1 bis 50:1, bevorzugt 0,5:1 bis 20:1, besonders bevorzugt 1:1 bis 15:1, ganz besonders bevorzugt 2:1 bis 12:1 zum schwerlöslichen kosmetischen Wirkstoff eingesetzt.

Der Gehalt an erfindungsgemäßem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholsulfonate, Fettalkoholethersulfate, Fettalkoholethersulfonate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Mundwässern, Haarwässern, Eau de Cologne, Eau de Toilette usw. Verwendung.

### Beschreibung der Solubilisierungsmethode:

Bei der Herstellung der Solubilisate für kosmetische Formulierugen können die Verbindungen der Formel I als 100%ige Substanz oder als wäßrige Lösung eingesetzt werden.

Üblicherweise wird der Solubilisator in Wasser gelöst und mit dem jeweils zu verwendenden schwerlöslichen kosmetischen Wirkstoff z.B. den o.g. etherischen Ölen bzw. Parfümölen intensiv vermischt, beispielsweise mittels eines Magnetrührers.

Es kann aber auch der zu verwendende schwerlösliche kosmetische Wirkstoff in einer Schmelze des Solubilisators gelöst werden und anschließend unter ständigem Rühren mit demineralisiertem Wasser versetzt werden.

### Solubilisatoren für pharmazeutische Anwendungen

Die beanspruchten Verbindungen eignen sich für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe oder Vitamine sowie Carotinoide enthalten. Insbesondere handelt es sich dabei um wäßrige Lösungen bzw. Solubilisate zur oralen oder parenteralen Applikation, wie z.B. Injektionslösungen zur intravenösen, intramuskulären oder subkutaner oder intraperitonealer Applikation.

Desweiteren eignen sich die beanspruchten Verbindungen zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die beanspruchten Verbindungen um einen schwerlöslichen Arzneistoff zu verarbeiten.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Die Verwendung der erfindungsgemäßen Verbindungen als Lösungsvermittler in pharmazeutischen Zubereitungen erfolgt beispielsweise in der Weise, daß der Wirkstoff in dem Solubilisator, gegebenenfalls unter Erwärmen, dispergiert oder gelöst wird und unter Rühren mit Wasser vermischt wird.

Eine andere Herstellvariante ist das Auflösen des Solubilisators in der wäßrigen Phase, gegebenenfalls unter leichtem Erwärmen und das anschließende Lösen des Wirkstoffs in der wäßrigen Solubilisatorlösung. Das gleichzeitige Auflösen von Solubilisator und Wirkstoff in der wäßrigen Phase ist ebenfalls möglich.

Gegenstand der Erfindung sind daher auch pharmazeutische Zubereitungen, die mindestens eine der Verbindungen der Formel I als Solubilisatoren enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um parenteral applizierbare Formulierungen handelt.

Der Gehalt an erfindungsgemäßem Solubilisator in der pharmazeutischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

### Solubilisatoren für Lebensmittelzubereitungen

Neben der Anwendung in der Kosmetik und Pharmazie eignen sich die erfindungsgemäßen Verbindungen der Formel I auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiel seien klare, mit Carotinoiden gefärbte Getränke genannt.

Gegenstand der Erfindung sind daher auch Lebensmittelzubereitungen, die mindestens eine der Verbindungen der Formel I als Solubilisatoren enthalten. Bevorzugt sind solche Zubereitungen, die neben dem Solubilisator ein in Wasser schwerlösliches oder wasserunlösliches Vitamin oder Carotinoid enthalten.

### Solubilisatoren für Pflanzenschutzzubereitungen

Die Anwendung der erfindungsgemäßen Verbindungen der Formel I als Solubilisatoren in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

In den folgenden Beispielen wird die Herstellung der polymerisierten Fettsäurederivate sowie deren Verwendung als Solubilisatoren näher erläutert.

### A) Herstellung der erfindungsgemäß verwendeten Ester von hydroxylierten Carbonsäuren

### Beispiel 1

### 12-Hydroxystearinsäure-methylpolyethylenglykol-(500)-monoester

a) 12-Hydroxystearinsäuremethylester
   In eine Lösung von 2200 g ca. 89%iger 12-Hydroxystearinsäure [Edenor® OSSG, Fa. Henkel; entsprechend ca. 1960 g (6,52 mol) reiner 12-HS] in 6000 ml Methanol wurden unter Rühren bei 40°C 268 g HCl-Gas innerhalb 1 h eingeleitet. Die Temperatur stieg auf 50°C an. Bei 50°C wurde 2 h nachgerührt. Durch anschließendes Einleiten von Stickstoff wurde das HCl-Gas weitgehend entfernt. Das Methanol wurde am Rotationsverdampfer unter Vakuum abdestilliert. Nach fraktionierter Kristallisation aus Aceton und anschließendem Trocknen erhielt man insgesamt 573 g (1,82 mol; 27,9% Ausbeute) 12-Hydroxystearinsäuremethylester mit einem Schmelzpunkt von 58°C und einer Reinheit von 98,2% (Gehalt nach GC).
b) 12-O-Tetrahydropyranyl-stearinsäuremethylester
   Eine Lösung von 500 g (1,59 mol) 12-Hydroxystearinsäuremethylester und 1,20 g p-Toluolsulfonsäure in 3500 ml CH₂Cl₂ wurde auf 0-5°C abgekühlt. Bei dieser Temperatur wurden unter Rühren 135 g (1,60 mol) 3,4-Dihydro-2H-pyran, gelöst in 250 ml CH₂Cl₂, innerhalb von 2,5 h zudosiert. Bei 2-5°C wurde insgesamt 2,5 h nachgerührt, wobei nach 1,5 h 1,35 g (1,6 mmol) 3,4-Dihydro-2H-pyran und nach 2 h 0,1 g p-Toluolsulfonsäure zugegeben wurden. Anschleßend wurden 2 g K₂CO₃ in Form einer konzentrierten wäßrigen Lösung zugegeben und kurz nachgerührt. Die organische Phase wurde zweimal mit Wasser, zweimal mit verdünntem Ammoniak sowie ein weiteres Mal mit Wasser gewaschen. Nach dem Trocknen der organischen Phase mit Natriumsulfat wurde das CH₂Cl₂ bei 25°C im Vakuum am Rotationsverdampfer abdestilliert. Ausbeute (roh): 655 g eines blaßgelben Öles.
   Je 130 g des Rohproduktes wurden in 200 ml n-Hexan gelöst und auf eine mit 1300 g Kieselgel gefüllte 4-L-Fritte aufgetragen. Eluiert wurde mit jeweils ca. 4 Liter n-Hexan sowie ca. 6 Liter n-Hexan/Methyl-t-butylether. Die Fraktionen, in welchen sich 12-O-THP-12-hydroxystearinsäuremethylester rein nachweisen ließ wurden vereinigt und am Rotationsverdampfer zur Trockene eingeengt. Man erhielt insgesamt 557,6 g (1,40 mol, 88%) eines farblosen Öls.
   1H-NMR-Spektrum: 270 MHz, CDCl_{3,} δ = 0,90 (t, 3H; CH₃-CH₂-), 1,1-1,95 (m, 34H), 2,31 (t, 2H; -CH₂-CO₂CH₃), 3,42-3,53 (m, 1H), 3,55-3,64 (m, 1H; -CH₂-CH(-O-THP)-CH₂-), 3,68 (s, 3H; -CO₂CH₃), 3,86-3,98 (m, 1H), 4,65 (m, 1H; O-CH-O)
c) 12-O-Tetrahydropyranyl-stearinsäure
   Eine Lösung von 100,0 g (0,251 mol) 12-O-THPstearinsäuremethylester und 28,2 g (0,503 mol) KOH in einem Lösungsmittel-Gemisch, bestehend aus 120 ml Dioxan und 100 ml Wasser, wurde unter Rühren auf 85°C erhitzt. Nach 1,5 h wurde das Reaktionsgemisch im Eisbad auf Raumtemperatur abgekühlt. Durch vorsichtige Zugabe einer gesättigten, wäßrigen CitronensäureLösung wurde ein pH von 4,5 eingestellt. Die wäßrige Lösung wurde anschließend 3 mal mit jeweils ca. 100 ml CH₂Cl₂ extrahiert. Das Lösungsmittel wurde am Rotationsverdampfer bei Raumtemperatur im Vakuum entfernt. Ausbeute: 95,7 g (0,248 mol, 98,8%) eines viskosen, farblosen Öls.
   1H-NMR-Spektrum: 270 MHz, CDCl₃, δ = 0,90 (t, 3H; CH₃-CH₂-), 1,1-1,95 (m, 34H), 2,34 (t, 2H; -CH₂-CO₂H), 3,42-3,57 (m, 1H), 3,58-3,64 (m, 1H; -CH₂-CH(-O-THP)-CH₂-), 3,85-3,98 (m, 1H), 4,65 (m, 1H; O-CH-O), 10,8-11,2 (br. s, 1H; -CO₂H).
d) 12-O-Tetrahydropyranyl-stearinsäure-methylpolyethylenglykol-(500)-monoester
   Eine bei Raumtemperatur hergestellte Lösung von 80,0 g (208 mmol) 12-O-THP-stearinsäure in 80 g CH₂Cl₂ wurde mit 3,05 g (25,0 mmol) 4-Dimethylamino-pyridin versetzt. Nach Abkühlen auf -3 bis +5°C mittels Eis/Kochsalzbad wurden 51,6 g (250 mmol) Dicyclohexylcarbodiimid als 50%ige Lösung in CH₂Cl₂, innerhalb einer Stunde bei dieser Temperatur zugetropft und 20 Minuten nachgerührt. 104,2 g (208 mmol) Methylpolyethylenglykol 500 (Pluriol® A 500 E, Fa. BASF) wurden als 50%ige Lösung in CH₂Cl₂ innerhalb einer Stunde bei 0°C zudosiert. Nach 20 Stunden Nachrührzeit wurde der ausgefallene Dicyclohexylharnstoff abgesaugt und das Filtrat 2 mal mit ca. 100 ml einer mit Salzsäure auf pH=3 angesäuerten, wäßrigen NaCl-Lösung gewaschen. Die organische Phase wurde mit Na₂SO₄ getrocknet und das Lösungsmittel am Rotationsverdampfer entfernt. Ausbeute: 178,3 g einer farblosen, wachsartigen Substanz.
   1H-NMR-Spektrum: 270 MHz, CDCl₃, δ = 0,90 (t, 3H; CH₃-CH₂-), 1,1-1,95 (m, 34H), 2,32 (t, 2H; -CH₂-CO₂-CH₂CH₂O-), 3,37 (s, 3H, CH₃-O-), 3,43-3,53 (m, 1H), 3,55-3,70 (m, ca. 47H; -CH₂-O-CH₂CH₂O-), 3,85-3,98 (m, 1H), 4,22 (t, 2H; -CO₂-CH₂CH₂O-), 4,64 (m, 1H; O-CH-O).
e) 12-Hydroxystearinsäure-methylpolyethylenglykol-(500)-monoester
   170 g (196 mmol) 12-O-THP-stearinsäure-methylpolyethylenglykol(500)-monoester wurden in 380 ml eines Ethanol/Wasser/Isopropanol (45:45:10)-Gemisches gelöst und ein pH-Wert von 2-3 durch Zugabe konzentrierter Salzsäure eingestellt. Man rührte zunächst 12 h bei 45°C und ließ dann über Nacht bei Raumtemperatur stehen. Man neutralisierte mit K₂CO₃ und engte am Rotationsverdampfer zur Trockene ein. Rohausbeute: ca. 130 g (ca. 85%) einer farblosen, wachsartigen Substanz. Das Rohprodukt wurde anschließend säulenchromatographisch aufgereinigt. Man erhielt ein farbloses Wachs mit einem Schmelzpunkt von 38-41°C. Ausbeute: 64%.
   1H-NMR-Spektrum: 400 MHz, CDCl₃, δ = 0,88 (t, ³J=7,0 Hz, 3H; CH₃-CH₂-), 1,3-1,4 (m, 26H; Alkylen-H), 1,62 (m, 2H; Alkylen-H) 2,32 (t, ³J=7,5 Hz, 2H; -CH₂-CO₂-CH₂CH₂O-), 3,38 (s, 3H; CH₃-OCH₂CH₂O-), 3,54-3,70 (m, ca. 48H; -CH₂-O-CH₂CH₂O-), 4,22 (t, ³J=4,8 Hz, 2H; -CO₂-CH₂CH₂O-).
   1H-NMR-Spektrum: 400 MHz, CDCl₃, [Derivatisierung mit Trichloracetylisocyanat (TAI)], δ = 0,88 (t, ³J=7,0 Hz, 3H; CH₃-CH₂-), 1,3 (m, 22H; Alkylen-H), 1,6 (m, 6H; Alkylen-H), 2,32 (t, ³J=7,6 Hz, 2H; -CH₂-CO₂-CH₂CH₂O-), 3,37 (s, 3H; CH₃-OCH₂CH₂O-), 3,55-3,70 (m, ca. 47H; -CH₂-O-CH₂CH₂O-), 4,21 (t, ³J=4,9 Hz, 2H; - CO₂-CH₂CH₂O-), 4,94 (quint., ³J=6,2 Hz, 1H; TAI-O-CH(CH₂-)₂).
   Das ¹H-NMR-Spektrum enthielt keine Signale einer mit Fettsäure veresterten Hydroxymethingruppe bei δ =4,90 ppm. Eine Veresterung von 12-Hydroxystearinsäure-Einheiten untereinander fand somit nicht statt.

### Beispiel 2

### 12-Hydroxystearinsäure-methylpolyethylenglykol-(750)-monoester

Die Herstellung wurde in Analogie zu der Synthesesequenz a) bis e) in Beispiel 1 durchgeführt. In Schritt d) wurde Methylpolyethylenglykol 750 (Pluriol® A 750 E) anstelle von Methylpolyethylenglykol 500 eingesetzt.

### Beispiel 3 (Vergleichsbeispiel)

### 12-Hydroxystearinsäure-methylpolyethylenglykol-(900)-monoester

Die Herstellung wurde in Analogie zu der Synthesesequenz a) bis e) in Beispiel 1 durchgeführt. In Schritt d) wurde Methylpolyethylenglykol 900 anstelle von Methylpolyethylenglykol 500 eingesetzt.

### Beispiel 4

### Veresterung von 12-Hydroxystearinsäure mit Methylpolyethylenglykol 470 (Molverhältnis 1:1)

63,0 g 12-Hydroxystearinsäure (Edenor® OSSG, Fa. Henkel, SZ = 181 mg KOH/g) wurden bei 80°C mit 96,6 g Methylpolyethylenglykol 470 (OHZ = 122 mg KOH/g) und 1,60 g 50%iger Hypophosphoriger Säure versetzt und 20 h bei 160°C unter Schutzgas gerührt. Das sich bildende Reaktionswasser wurde abdestilliert.
Ausbeute: 152,2 g
Säurezahl (SZ): 6,3 mg KOH/g; OH-Zahl (OHZ): 58 mg KOH/g; Verseifungszahl (VZ): 92 mg KOH/g.

### Beispiel 5

### Veresterung von 12-Hydroxystearinsäure mit Methylpolyethylenglykol 500 (Molverhältnis 1:1)

60,0 g 12-Hydroxystearinsäure (Edenor® OSSG, Fa. Henkel, SZ = 181 mg KOH/g) wurden bei 80°C mit 100,0 g Methylpolyethylenglykol 500 (Pluriol® A 500 E, Fa. BASF; OHZ = 109 mg KOH/g) und 1,60 g 50%iger Hypophosphoriger Säure versetzt, und 20 h bei 160°C unter Schutzgas gerührt. Das sich bildende Reaktionswasser wurde abdestilliert.
Ausbeute: 152 g
Säure-Zahl (SZ): 6,9 mg KOH/g; OH-Zahl (OHZ): 54 mg KOH/g; Verseifungszahl (VZ): 89 mg KOH/g;

### Beispiel 6

### Veresterung von 12-Hydroxystearinsäure mit Methylpolyethylenglykol 750 (Molverhältnnis 1:1)

90,0 g 12-Hydroxystearinsäure (Edenor® OSSG, Fa. Henkel) wurden bei 80°C mit 218,7 g Methylpolyethylenglykol 750 (Pluriol® A 750 E, Fa. BASF; OHZ = 77,0 mg KOH/g) und 3,10 g 50%iger Hypophosphoriger Säure versetzt und 21 h bei 180°C unter Schutzgas gerührt. Das sich bildende Reaktionswasser wurde abdestilliert.
Ausbeute: 303 g
SZ: 3,6 mg KOH/g; OHZ: 43 mg KOH/g; VZ: 60 mg KOH/g.

### Beispiel 7

### Veresterung von 12-Hydroxystearinsäure mit einem Gemisch von Methylpolyethylenglykol 500 und Methylpolyethylenglykol 750 (Molverhältnnis 1,5:0,6:0,4)

79,1 g 12-Hydroxystearinsäure (Edenor® OSSG, Fa. Henkel, SZ = 181 mg KOH/g) wurden bei 80°C mit 51,0 g Methylpolyethylenglykol 500 (Pluriol® A 500 E, Fa. BASF; OHZ = 112 mg KOH/g) sowie 51,0 g Methylpolyethylenglykol 750 (Pluriol® A 750 E, Fa. BASF; OHZ = 77,0 mg KOH/g) versetzt. Nach Zugabe von 1,81 g 50%iger Hypophosphoriger Säure wurde 20 h bei 165°C unter Schutzgas gerührt. Das sich bildende Reaktionswasser wurde abdestilliert.
Ausbeute: 172,7 g
SZ: 7,8 mg KOH/g; OHZ: 33 mg KOH/g; VZ: 91 mg KOH/g.

### Beispiel 8

### Veresterung von 12-Hydroxystearinsäure mit einem Gemisch von Methylpolyethylenglykol 500 und Methylpolyethylenglykol 750 (Molverhältnnis 2:0,6:0,4)

93,0 g 12-Hydroxystearinsäure (Edenor® OSSG, Fa. Henkel, SZ = 181 mg KOH/g) wurden bei 80°C mit 45,0 g Methylpolyethylenglykol 500 (Pluriol® A 500 E, Fa. BASF; OHZ = 109 mg KOH/g) sowie 45,0 g Methylpolyethylenglykol 750 (Pluriol® A 750 E, Fa. BASF; OHZ = 77,0 mg KOH/g) versetzt. Nach Zugabe von 1,83 g 50%iger Hypophosphoriger Säure wurde 20 h bei 165°C unter Schutzgas gerührt. Das sich bildende Reaktionswasser wurde abdestilliert.
Ausbeute: 173,8 g
SZ: 9,3 mg KOH/g; OHZ: 28 mg KOH/g; VZ: 105 mg KOH/g.

### Beispiel 9 (Vergleichsbeispiel)

### Veresterung von 12-Hydroxystearinsäure mit Methylpolyethylenglykol 900 (Molverhältnis 1:1)

39,0 g 12-Hydroxystearinsäure (Edenor® OSSG, Fa. Henkel) wurden bei 80°C mit 117,0 g Methylpolyethylenglykol 900 (OHZ = 62 mg KOH/g) und 1,56 g 50%iger Hypophosphoriger Säure versetzt und 20 h bei 160°C unter Schutzgas gerührt. Das sich bildende Reaktionswasser wurde abdestilliert.
Ausbeute: 150 g
SZ: 5,9 mg KOH/g; OHZ: 36 mg KOH/g; VZ: 60 mg KOH/g.

### Beispiel 10

### Veresterung von 12-Hydroxystearinsäure mit Methylpolyethylenglykol 900 (Molverhältnis 2:1)

Eine Schmelze von 90,0 g Methylpolyethylenglykol 900 (OHZ = 62 mg KOH/g) wurde bei 80°C mit 1,50 g 50%iger Hypophosphoriger Säure versetzt. Nach dem Erwärmen unter Schutzgas auf 165°C wurden unter Rühren 60,0 g einer ca. 90°C warmen 12-Hydroxystearinsäure-Schmelze (Edenor® OSSG, Fa. Henkel) innerhalb von 8 h zugetropft. Hierbei wurde die Temperatur langsam bis auf 180°C erhöht. Weitere 20 h wurde bei 180°C gerührt. Das sich bildende Reaktionswasser wurde abdestilliert.
Ausbeute: 137 g
SZ: 5,7 mg KOH/g; OHZ: 40 mg KOH/g; VZ: 81 mg KOH/g.

### Beispiel 11 Vergleichsbeispiel

### Veresterung von 12-Hydroxystearinsäure mit Methylpolyethylenglykol 1300 (Molverhältnis 1:1)

45,0 g 12-Hydroxystearinsäure (Edenor® OSSG, Fa. Henkel) wurden bei 80°C mit 195 g Methylpolyethylenglykol 1300 (OHZ = 44 mg KOH/g) und 2,4 g 50%iger Hypophosphoriger Säure versetzt und 6 h bei 170°C unter Schutzgas gerührt. Das sich bildende Reaktionswasser wurde abdestilliert. Für weitere 4 h wurde die Temperatur auf 180°C erhöht. Nach dem Abkühlen auf 80°C wurden je 12,3 g Aluminiumoxid und Ambosol sowie 0,6 g Hyflow zugegeben, 1 h bei 80°C gerührt und noch heiß über eine Drucknutsche filtriert.
Ausbeute: 221,6 g
SZ: 2,2 mg KOH/g.

### Beispiel 12

### Veresterung einer Mischung von Dimerfettsäure und 12-Hydroxystearinsäure mit Methylpolyethylenglykol 500 (Molverhältnis 1:0,9:2,1)

Eine Mischung von 29,0 g 12-Hydroxystearinsäure (Edenor OSSG, Fa. Henkel) und 47,6 g Dimerfettsäure (Pripol 1009, Fa. Unichema, SZ = 193 mg KOH/g) wurden bei 80°C mit 101,6 g Methylpolyethylenglykol 500 (Pluriol A 500 E, Fa. BASF, OHZ = 109 mg KOH/g) und 1,78 g 50%iger Hypophosphoriger Säure versetzt und 20 h bei 170°C unter Schutzgas gerührt. Das sich bildende Reaktionswasser wurde abdestilliert.
Ausbeute: 163,9 g
SZ: 9,7 mg KOH/g; OHZ: 9 mg KOH/g; VZ: 92 mg KOH/g.

### Beispiel 13a (Vorstufe von Beispiel 13b und 13c)

### Veresterung von 12-Hydroxystearinsäure mit Dimerfettsäure (Molverhältnis 1:0,9)

65,3 g 12-Hydroxystearinsäure (Edenor® OSSG, Fa. Henkel) wurden bei 80°C mit 107,5 g (Pripol® 1009, Fa. Unichema, SZ = 193 mg KOH/ g) und 1,73 g 50%iger Hypophosphoriger Säure versetzt und 14,5 h bei 170°C unter Schutzgas gerührt. Das sich bildende Reaktionswasser wurde abdestilliert.
Ausbeute: 166 g
SZ: 139 mg KOH/g; OHZ: 3 mg KOH/g; VZ: 202 mg KOH/g.

### Beispiel 13b

### Veresterung einer Mischung von Dimerfettsäure und 12-Hydroxystearinsäure mit Methylpolyethylenglykol 500 (Molverhältnis 1:0,9:2,1)

54,5 g des Vorkondensates aus Beispiel 13a wurden bei 80°C mit 74,0 g Methylpolyethylenglykol 500 (Pluriol® A 500 E, Fa. BASF, OHZ = 109 mg KOH/g) und 1,28 g 50%iger Hypophosphoriger Säure versetzt und 12 h bei 170°C unter Schutzgas gerührt. Das sich bildende Reaktionswasser wurde abdestilliert.
Ausbeute: 121,5 g
SZ: 7,7 mg KOH/g; OHZ: 6 mg KOH/g; VZ: 94 mg KOH/g.

### Beispiel 13c Vergleichsbeispiel

### Veresterung einer Mischung von Dimerfettsäure und 12-Hydroxystearinsäure mit Methylpolyethylenglykol 900 (Molverhältnis 1:0,9:2)

41,3 g des Vorkondensates aus Beispiel 13a wurden bei 80°C mit 94,3 g Methylpolyethylenglykol 900 (OHZ = 62 mg KOH/g) und 1,36 g 50%iger Hypophosphoriger Säure versetzt und 20 h bei 170°C unter Schutzgas gerührt. Das sich bildende Reaktionswasser wurde abdestilliert.
Ausbeute: 128,1 g
SZ: 6,7 mg KOH/g; OHZ: 4 mg KOH/g; VZ: 68 mg KOH/g.

### Beispiel 14 Vergleichsbeispiel

### Veresterung einer Mischung von Stearinsäure und 12-Hydroxystearinsäure mit Methylpolyethylenglykol 900 (Molverhältnis 0,75:1:0,95)

Ein Schmelze, bestehend aus 26,9 g Stearinsäure (Edenor C18 98/100, Fa. Henkel; SZ=200 mg KOH/g) und 38,8 g 12-Hydroxystearinsäure (Edenor OSSG, Fa. Henkel), wurde bei 80-90°C mit 106,3 g Methylpolyethylenglykol 900 (OHZ = 62 mg KOH/g) und 1,72 g 50%iger Hypophosphoriger Säure versetzt und 20 h bei 180°C unter Schutzgas gerührt. Das sich bildende Reaktionswasser wurde abdestilliert.
Ausbeute: 163,6 g
SZ: 12,3 mg KOH/g; OHZ: 8 mg KOH/g; VZ: 78 mg KOH/g.

### Beispiel 15a (Vorstufe für Beispiel 15b)

### Veresterung von 12-Hydroxystearinsäure mit Laurinsäure (Molverhältnis 1:0,75)

120,0 g 12-Hydroxystearinsäure (Edenor® OSSG, Fa. Henkel) wurden bei 80°C mit 59,2 g Laurinsäure und 1,79 g 50%iger Hypophosphoriger Säure versetzt und 4 h bei 165-170°C unter Schutzgas gerührt. Das sich bildende Reaktionswasser wurde abdestilliert.
Ausbeute: 172,6 g
SZ: 118,4 mg KOH/g; OHZ: 8 mg KOH/g; VZ: 229 mg KOH/g.

### Beispiel 15b Vergleichsbeispiel

### Veresterung von 12-Hydroxystearinsäure mit Laurinsäure und mit Methylpolyethylenglykol 900 (Molverhältnis 1:0,75:0,9)

39,4 g des Vorkondensates aus Beispiel 15a wurden bei 80°C mit mit 71,0 g Methylpolyethylenglykol 900 (OHZ = 62 mg KOH/g) und 1,10 g 50%iger Hypophosphoriger Säure versetzt und 20 h bei 170-180°C unter Schutzgas gerührt. Das sich bildende Reaktionswasser wurde abdestilliert.
Ausbeute: 102 g
SZ: 7,9 mg KOH/g; OHZ: 5 mg KOH/g; VZ: 69 mg KOH/g.

### Beispiel 16 Vergleichsbeispiel

### Veresterung einer Mischung von Laurinsäure und 12-Hydroxystearinsäure mit Methylpolyethylenglykol 1300 (Molverhältnis 0,75:1:1)

Eine Mischung von 28,4 g 12-Hydroxystearinsäure (Edenor OSSG, Fa. Henkel), 14,0 g Laurinsäure und 1,60 g 50%iger Hypophosphoriger Säure wurde bei 80°C mit 114,9 g Methylpolyethylenglykol 1300 (OHZ = 44 mg KOH/g) versetzt und 25 h bei 170°C unter Schutzgas gerührt. Das sich bildende Reaktionswasser wurde abdestilliert.
Ausbeute: 143 g
SZ: 6,8 mg KOH/g; OHZ: 22 mg KOH/g; VZ: 65 mg KOH/g.

### Beispiel 17 Vergleichsbeispiel

### Veresterung einer Mischung von Laurinsäure und 12-Hydroxystearinsäure mit Methylpolyethylenglykol 1300 (Molverhältnis 0,45:1:0,65)

Eine Mischung 38,0 g 12-Hydroxystearinsäure (Edenor OSSG, Fa. Henkel) und 11,5 g Laurinsäure wurden bei 80°C mit 102,6 g Methylpolyethylenglykol 1300 (OHZ = 44 mg KOH/g) und 1,50 g 50%iger Hypophosphoriger Säure versetzt und 21 h bei 165-180°C unter Schutzgas gerührt. Das sich bildende Reaktionswasser wurde abdestilliert.
Ausbeute: 141 g
SZ: 8,7 mg KOH/g; OHZ: 9 mg KOH/g; VZ: 75 mg KOH/g.

### B) Pharmazeutische und kosmetische Formulierungen

### Beispiel 18

### Diazepam Injektionslösung

400 mg 12-Hydroxystearinsäure-MPG-500-Ester 1:1 (hergestellt gemäß Beispiel 5) wurden in 1578 mg Aqua bidest gelöst. Anschließend wurden 10 mg Diazepam zu der Solubilisatorlösung gegeben und gerührt bis der Arzneistoff gelöst war. Die Lösung wurde mit 2 mg Natriumdisulfit und 10 mg Benzylalkohol konserviert und nach üblichen Methoden sterilfiltriert und in Injektionsfläschen gefüllt.

### Beispiel 19

### 17-β-Estradiol Gelatine Kapseln

100 mg 17-β-Estradiol wurden mit 10 g 12-HydroxystearinsäureMPG-500-Ester 1:1 (hergestellt gemäß Beispiel 5) und 80 g geschmolzenem PEG 6000 sowie 10 g Ethanol gemischt und anschließend direkt in flüssiger Form in Kapseln gefüllt.

### Beispiel 20

### Orale Ciclosporin Formulierung (flüssig befüllte Kapsel)

100 g Ciclosporin A wurden in 770 g 12-HydroxystearinsäureMPG-500-Ester 1:1 (hergestellt gemäß Beispiel 5), 100 ml Ethanol und 75 ml Propylenglykol gelöst und die viskose klare Lösung wurde anschließend in Kapseln abgefüllt. Diese Lösung war unbegrenzt mit Wasser verdünnbar.

### Beispiel 21

### Diazepam Emulsion zur parenteralen Applikation

160 g 12-Hydroxystearinsäure-MPG-500-Ester 1:1 (hergestellt gemäß Beispiel 5) wurden in 660 g Aqua bidest gelöst. Das Diazepam (10 g) wurde in einer 1:1 Mischung aus Sojaöl und Miglyol Öl (Ölphase beträgt 200 g) dispergiert. Zusätzlich wurden 10 g Sojalecithin eingesetzt, das in der Ölphase gelöst wurde. Die beiden Phasen wurden vordispergiert und anschließend per Hochdruckhomogenisation emulgiert.

### Beispiel 22

### 17-β-Estradiol Tablette

10 g 17-β-Estradiol wurden mit 50 g 12-Hydroxystearinsäure-MPG-500-Ester 1:1 (hergestellt gemäß Beispiel 5) geschmolzen. Die Schmelze wurde auf 940 g Ludipress gezogen. Das Granulat wurde anschließend mit 0,5 g Mg-stearat gemischt und anschließend tablettiert.

### Beispiel 23

### Diazepam-haltiges Pulver

Diazepam und 12-Hydroxystearinsäure-MPG-500-Ester 1:1 (hergestellt gemäß Beispiel 5) als Solubilisator wurden in Ethanol gelöst. Anschließend wurde Sorbitol als Trägerstoff hinzugefügt und ebenfalls gelöst. Das Lösungsmittel wurde entfernt und die Mischung im Vakuum getrocknet.

### Beispiel 24

### Sonnenschutzmittel

25 g 12-Hydroxystearinsäure-MPG-500-Ester 1:1 (hergestellt gemäß Beispiel 5) wurden bei ca. 60 °C geschmolzen und 2,5 g Uvinul T 150 wurden in der Schmelze gelöst. Anschließend wurde eine auf 60°C erwärmte Mischung von 62,5 g Aqua bidest und 10 g Glycerol vorsichtig unter Rühren hinzugetropft. Es entstand eine klare Lösung, die nach Erkalten auf Raumtemperatur in ein geeignetes Behältnis abgefüllt wurde.

### C) Anwendungsbeispiele

### Beispiel 25

### Solubilisierende Wirkung am Beispiel 17-β-Estradiol, Sulfathiazol und Clotrimazol

Es wurden 20%ige Solubilisatorlösungen eingesetzt. Die Ester der hydroxylierten Carbonsäuren wurden unter leichtem Erwärmen (Temp. bis 65 °C) geschmolzen und der Arzneistoff zur Schmelze hinzugegeben. Danach wurde der Phosphatpuffer pH 7,0 (USP XXIII) in kleinen Anteilen hinzugefügt. Es wurde bei Raumtemperatur gerührt bis die Sättigungskonzentration des Arzneistoffs erreicht war.

| Verbindung | Sulfathiazol | 17-β-Estradiol | Clotrimazol |
|---|---|---|---|
| Phosphatpuffer pH 7,0 | 0,07 | 0,0 | 0,0 |
| Sorbitanfettsäureester (Tween® 80) | 0,7 | 0,09 | 0,03 |
| Ethoxyliertes Ricinusöl (Cremophor® EL) | 0,7 | 0,06 | 0,01 |
| 12-Hydroxystearinsäure-MPG-500-Ester (Beispiel 5) | 0,43 | 0,35 | 0,18 |
| 12-Hydroxystearinsäure-MPG-470-Ester (Beispiel 4) | 0,44 | 0,36 | 0,18 |
| 12-Hydroxystearinsäure-MPG-750-Ester (Beispiel 2) | 0,81 | 0,30 | 0,31 |
| 12-Hydroxystearinsäure-MPG-750-Ester (Beispiel 6) | 0,54 | 0,31 | 0,20 |
| 12-Hydroxystearinsäure-MPG-500-Ester (Beispiel 1) | 0,95 | 0,40 | 0,37 |

Die Zahlenangaben beziehen sich auf die Menge an solubilisiertem Arzneistoff in Gewichtsprozent.

### Beispiel 26:

### Hämolyseaktivität

In einem RBC-Test (Red Blood Cell) an Humanerythrozyten wurde die hämolytische Aktivität der beanspruchten Verbindungen getestet.

Die Inkubationszeit betrug 60 min bei Raumtemperatur. Die geringe hämolytische Aktivität der 1 %-igen Lösungen ist ersichtlich.

| Verbindung | Hämolyse der 1 %-igen Lösungen in Phosphatpuffer¹⁾ |
|---|---|
| Phosphatpuffer pH 7,0 | 0% |
| Sorbitanfettsäureester (Tween 80) | 4% |
| Solutol® HS 15 | 1% |
| Ethoxyliertes Ricinusöl (Cremophor® EL) | 0% |
| 12-Hydroxystearinsäure-MPG-500-Ester (Beispiel 5) | 3% |
| 12-Hydroxystearinsäure-MPG-470-Ester (Beispiel 4) | 0% |
| 12-Hydroxystearinsäure-MPG-750-Ester (Beispiel 2) | 1% |
| 12-Hydroxystearinsäure-MPG-750-Ester (Beispiel 6) | 0% |
| 12-Hydroxystearinsäure-MPG-500-Ester (Beispiel 1) | 0% |

| | |
|---|---|
| ¹⁾ Die %-Angaben beziehen sich auf die photometrisch gemessenen Hämolysewerte, bezogen auf die Maximalwerte bei vollständig induzierter Hämolyse. | |

### Beispiel 27

### Verträglichkeit am Hund

Nach intravenöser Injektion einer 5 %-igen wäßrigen Lösung der beanspruchten Verbindungen am Hund wurde die Bluthistaminauschüttung verfolgt. Die beanspruchten Substanzen zeigten eine geringere Erhöhung des Bluthistaminspiegels als die bekannten Solubilisatoren:

| Verbindung | 5 min. vor Applikation | 5 min. nach Applikation | 15 min. nach Applikation |
|---|---|---|---|
| 12-Hydroxystearinsäure-MPG-500-Ester (Beispiel 1) | 4 | 28 | 6 |
| 12-Hydroxystearinsäure-MPG-750-Ester (Beispiel 2) | 3 | 23 | 3 |
| 12-Hydroxystearinsäure- | 5 | 6 | 7 |
| MPG-500-Ester (Beispiel 5) | | | |
| | | | |

| Vergleichsbeispiel | | | |
|---|---|---|---|
| | | | |
| Sorbitanfettsäureester (Tween® 80) | 3 | 14142 | 58065 |
| Solutol® HS 15 | 5 | 138 | 220 |
| 12-Hydroxystearinsäure-MPG-900-Ester (Beispiel 3) | 4 | 5731 | 4682 |
| 12-Hydroxystearinsäure-MPG-900-Ester (Beispiel 9) | 7 | 1484 | 918 |
| 12-Hydroxystearinsäure-MPG-1300-Ester (Beispiel 11) | 10 | 11746 | 6774 |

Die Zahlenangaben stellen Bluthistaminspiegel in ng/ml dar.

## Patentansprüche

1. Verwendung von Estern der 12-Hydroxystearinsäure gemäß der allgemeinen Formel I, 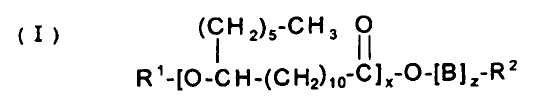 in der die Substituenten und Variablen unabhängig voneinander folgende Bedeutung haben:
R¹ Wasserstoff, C₁₂-C₂₂-Acyl, 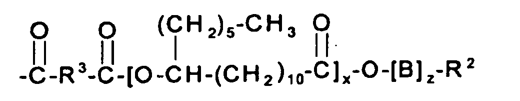
R² Methyl;
R³ Rest einer dimerisierten Fettsäure;
B -CH₂-CH₂-O-;
x 1 bis 2;
z 10 bis 18.
als Solubilisatoren.

2. Verwendung von Estern der 12-Hydroxystearinsäure gemäß Anspruch 1 als Solubilisatoren in pharmazeutischen und kosmetischen Zubereitungen.

3. Verwendung von Estern der 12-Hydroxystearinsäure gemäß Anspruch 2 als Solubilisatoren in Lebensmittelzubereitungen.

4. Pharmazeutische Zubereitungen, enthaltend mindestens eine der hydroxylierten Carbonsäureester gemäß Anspruch 1 als Solubilisator.

5. Pharmazeutische Zubereitungen nach Anspruch 4, enthaltend zusätzlich mindestens einen in Wasser schwerlöslichen oder wasserunlöslichen pharmazeutischen Wirkstoff.

6. Pharmazeutische Zubereitungen nach einem der Ansprüche 4 oder 5, **dadurch gekennzeichnet, daß** es sich um parenteral applizierbare Darreichungsformen handelt.

7. Kosmetische Zubereitungen, enthaltend mindestens eine der hydroxylierten Carbonsäureester gemäß Anspruch 1 als Solubilisator.

8. Kosmetische Zubereitungen nach Anspruch 7, enthaltend zusätzlich mindestens einen in Wasser schwerlöslichen oder wasserunlöslichen kosmetischen Wirkstoff.

9. Lebensmittelzubereitungen, enthaltend mindestens eine der hydroxylierten Carbonsäureester gemäß Anspruch 1 als Solubilisator.

10. Lebensmittelzubereitungen nach Anspruch 9, enthaltend zusätzlich mindestens ein in Wasser schwerlösliches oder wasserunlösliches Vitamin oder Carotinoid.

## Claims

1. The use of esters of 12-hydroxystearic acid of the formula I 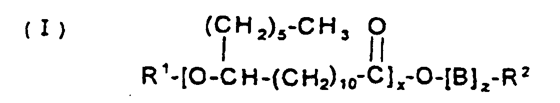 in which the substituents and variables independently of one another have the following meanings:
R¹ is hydrogen, C₁₂-C₂₂-acyl, 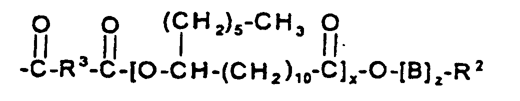
R² is methyl;
R³ is the radical of a dimerized fatty acid;
B is -CH₂-CH₂-O-;
x is 1 to 2;
z is 10 to 18
as solubilizers.

2. The use of esters of 12-hydroxystearic acid as claimed in claim 1.

3. The use of esters of 12-hydroxystearic acid as claimed in claim 2 as solubilizers in food preparations.

4. A pharmaceutical preparation comprising at least one of the hydroxylated carboxylic esters as claimed in claim 1 as solubilizer.

5. A pharmaceutical preparation as claimed in claim 4, comprising, in addition, at least one pharmaceutical active ingredient which is insoluble or virtually insoluble in water.

6. A pharmaceutical preparation as claimed in either of claims 4 and 5 in a parenterally administerable presentation.

7. A cosmetic preparation comprising at least one of the hydroxylated carboxylic esters as claimed in claim 1 as solubilizer.

8. A cosmetic preparation as claimed in claim 7, comprising, in addition, at least one cosmetic active ingredient which is insoluble or virtually insoluble in water.

9. A food preparation comprising at least one of the hydroxylated carboxylic esters as claimed in claim 1 as solubilizer.

10. A food preparation as claimed in claim 9, comprising, in addition, at least one vitamin or carotenoid which is insoluble or virtually insoluble in water.

## Revendications

1. Utilisation d'esters de l'acide 12-hydroxystéarique répondant à la formule générale I : 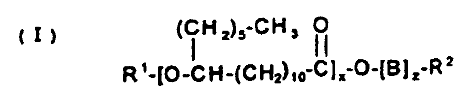 dans laquelle les substituants et variables ont, indépendamment les uns des autres, la signification suivante :
R¹ est de l'hydrogène, un acyle en C₁₂-C₂₂, 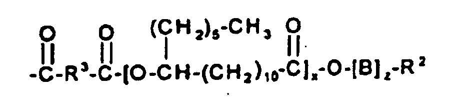
R² est du méthyle,
R³ est un radical d'un acide gras dimérisé,
B est -CH₂-CH₂-O-,
x a une valeur de 1 à 2,
z a une valeur de 10 à 18,
comme solubilisateurs.

2. Utilisation d'esters de l'acide 12-hydroxystéarique suivant la revendication 1, comme solubilisateurs dans des compositions pharmaceutiques et cosmétiques.

3. Utilisation d'esters de l'acide 12-hydroxystéarique suivant la revendication 2, comme solubilisateurs dans des compositions alimentaires.

4. Compositions pharmaceutiques, contenant au moins un des esters d'acide carboxylique hydroxylés suivant la revendication 1, comme solubilisateur.

5. Compositions pharmaceutiques suivant la revendication 4, contenant en supplément au moins une substance active pharmaceutique insoluble dans l'eau ou difficilement soluble dans celle-ci.

6. Compositions pharmaceutiques suivant l'une des revendications 4 et 5, **caractérisées en ce qu'**il s'agit de formes d'administration applicables par voie parentérale.

7. Compositions cosmétiques contenant au moins un des esters d'acide carboxylique hydroxylés suivant la revendication 1, comme solubilisateur.

8. Compositions cosmétiques suivant la revendication 7, contenant en supplément au moins une substance active cosmétique insoluble dans l'eau ou difficilement soluble dans celle-ci.

9. Compositions alimentaires contenant au moins un des esters d'acide carboxylique hydroxylés suivant la revendication 1, comme solubilisateur.

10. Compositions alimentaires suivant la revendication 9, contenant en supplément au moins un caroténoïde ou vitamine insoluble dans l'eau ou difficilement soluble dans celle-ci.
